# EUROPEAN PATENT SPECIFICATION

(11) **EP 2 988 727 B1**
(45) Date of publication and mention of the grant of the patent: **01.05.2019**
(21) Application number: 14722990.0
(22) Date of filing: 17.04.2014
(51) Int. Cl.: A61K 9/20, A61K 9/00, A61K 31/496

(54) **PHARMACEUTICAL COMPOSITION COMPRISING AN ATYPICAL ANTIPSYCHOTIC AGENT AND METHOD FOR THE PREPARATION THEREOF**
PHARMAZEUTISCHE ZUSAMMENSETZUNG MIT EINEM ATYPISCHEN ANTIPSYCHOTISCHEN WIRKSTOFF UND VERFAHREN ZUR HERSTELLUNG DAVON
COMPOSITION PHARMACEUTIQUE COMPRENANT UN AGENT ANTIPSYCHOTIQUE ATYPIQUE ET PROCÉDÉ DE PRÉPARATION CORRESPONDANT

(30) Priority: 22.04.2013 GR 20130100249
(43) Date of publication of application: 02.03.2016
(73) Proprietor: Pharmathen S.A., 15351 Pallini Attikis (GR)
(72) Inventor: KARAVAS, Evangelos, GR-153 51 Pallini Attikis (GR); KOUTRIS, Efthimios, GR-153 51 Pallini Attikis (GR); SAMARA, Vasiliki, GR-153 51 Pallini Attikis (GR); KOUTRI, Ioanna, GR-153 51 Pallini Attikis (GR); KALASKANI, Anastasia, GR-153 51 Pallini Attikis (GR); GOTZAMANIS, George, GR-153 51 Pallini Attikis (GR); KAKOURIS, Andreas, GR-153 51 Pallini Attikis (GR)
(86) International application number: PCT/EP2014/001040
(87) International publication number: WO 2014/173515

(56) References cited:
- WO-A1-2013/100878
- WO-A1-2014/037022
- CN-A- 102 451 162
- US-A1- 2009 208 576
- US-B1- 7 811 604

## Description

### TECHNICAL FIELD OF THE INVENTION

The present invention relates to a stable orally dispersible pharmaceutical formulation comprising a therapeutically effective quantity of the atypical antipsychotic agent Aripiprazole or pharmaceutical acceptable salt and a method for the preparation thereof.

### BACKGROUND OF THE INVENTION

Atypical antipsychotics (also known as second generation antipsychotics) are a class of prescription medications used for the treatment of psychiatric conditions. Said drugs have found favor among clinicians and gradually are replacing the typical antipsychotic agents. Atypical antipsychotics are now considered to be first line treatment for schizophrenia.

Both generations of medication tend to block receptors in the brain's dopamine pathways, but atypical differ from typical antipsychotics in that they are less likely to cause extrapyramidal motor control disabilities in patients, which include unsteady Parkinson's disease-type movements, body rigidity and involuntary tremors. These abnormal body movements can become permanent even after medication is stopped.

Aripiprazole works in a slightly different way to other antipsychotic medicines. It acts on various receptors in the brain, particularly dopamine receptors and serotonin receptors. Dopamine and serotonin are natural compounds called neurotransmitters, and are involved in transmitting messages between brain cells. Psychotic illness is considered to be caused by disturbances in the activity of neurotransmitters (mainly dopamine) in the brain. Aripiprazole is thought to work mainly by stabilizing the dopamine activity in the brain. It is considered to be a dopamine D₂receptor partial agonist. A dopamine D₂receptor partial agonist has affinity toward the dopamine D₂receptor and an intrinsic activity that is less than the activity of the endogenous full dopamine agonist, that is, it can bind to the dopamine D₂receptor and cause a similar set of reaction but the magnitude of the reaction is smaller. As a consequence Aripiprazole is more safe and tolerable in comparison to the existent typical and atypical antipsychotics.

People with schizophrenia may experience 'positive symptoms' (such as hallucinations, delusions and hostility) and/or 'negative symptoms' (such as lack of emotion and social isolation). The positive symptoms are thought to be due to overactivity of dopamine in certain areas of the brain. Aripiprazole blocks the dopamine receptors in these areas and so prevents the overactivity. This helps control the positive symptoms of the disease. The negative symptoms, as well as cognitive symptoms such as memory loss and poor attention, are considered to be due to underactivity of dopamine in other areas of the brain. In these areas, Aripiprazole stimulates the dopamine receptors, mimics the activity of dopamine of the brain and so improves their activity. Thus, the negative and cognitive symptoms of the illness are improved.

The chemical name of Aripiprazole is 7-{4-[4-(2,3-dichlorophenyl)piperazin-1-yl]butoxy]-3,4-dihydroquinolin-2(1H)-one and itsmolecular formula is C₂₃H₂₇Cl₂N₃O₂ corresponding to a molecular weight of 448.385. It is a white to off-white crystalline powder freely soluble in N,N-dimethyl formamide, dichloromethane and practically insoluble in water.

WO2008/020820 A1 discloses a pharmaceutical composition comprising Aripiprazole, at least a filler selected from the group consisting of mannitol, isomalt and sorbitol, at least a disintegrant selected from the group consisting of crospovidone, croscarmellose sodium and sodium starch glycolate, a pharmaceutical acceptable binder and a pharmaceutical acceptable lubricant.

EP 1542668 B1 discloses a pharmaceutical formulation comprising Aripiprazole and a substituted beta-cyclodextrin.

US2007/148100 A1 discloses a stable nanoparticulate Aripiprazole composition comprising: a) particles of Aripiprazole having an average effective particle size of less than about 2000nm; and b) at least one surface stabilizer.

US2009/0208576 discloses a pharmaceutical composition comprising an acid labile active ingredient with an organic stabilizing agent in a core, which core is coated with an intermediate layer of an organic stabilizer and a water insoluble polymer and an outer enteric coating layer.

Although each of the patents above represents an attempt to overcome the stability and solubility problems associated with pharmaceutical compositions comprising Aripiprazole, there still exists a need for a stable pharmaceutical composition which avoids such problems.

### SUMMARY OF THE INVENTION

It is, therefore, an object of the present invention to provide a stable solid dosage composition dispersible in the oral cavity containing an atypical antipsychotic agent and in particular Aripiprazole or pharmaceutical acceptable salt as the active ingredient, which overcomes the deficiencies of the prior art.

It is another object of the present invention to provide an orally disintegrating tablet comprising Aripiprazole in order to enhance both drug efficacy and patient compliance. Further object of the present invention is to provide a stable solid dosage formulation for oral administration containing Aripiprazole that overcomes the low water solubility of the active ingredient and has acceptable pharmacotechnical properties.

A major object of the present invention is the selection of the optimal combination of pharmaceutical acceptable excipients and method of preparation in order to achieve the appropriate dissolution profile and stability for the finished dosage form. Said dosage form affords predictable and reproducible drug release rates in order to achieve better treatment to a patient.

Further object of the present invention is to provide an orally disintegrating tablet comprising Aripiprazole or pharmaceutical acceptable salt as an active ingredient, which is bioavailable and with sufficient self-life.

Present claim 1 discloses an orally dispersible pharmaceutical composition comprising Aripiprazole or a pharmaceutical acceptable salt thereof as the active ingredient and an effective amount of maltose and microcrystalline cellulose wherein the ratio of maltose to microcrystalline cellulose is in the range of 1:1 to 1:4 by weight.

A further approach of the present invention is to provide an orally disintegrating tablet comprising Aripiprazole which is manufactured through a fast, simple and cost-effective process.

According to another embodiment of the present invention, a process for the preparation of a stable, orally disintegrating tablet containing the atypical antipsychotic agent Aripiprazole is provided, which comprises the following steps:
- weighing the raw materials and the active substance that will form the internal phase;
- dry blending of the ingredients of the internal phase;
- kneading the above blend with water;
- drying of the wet mass, preferably at 40°C;
- sizing of the granules obtained after drying;
- mixing with the excipients of the external phase; and
- compressing the above blend into tablets.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

The terms "orally disintegrating tablet" and "orally dispersible tablet" are used interchangeably unless otherwise stated.

As already mentioned the main object of the present invention is to provide an orally disintegrating tablet of Aripiprazole or pharmaceutical acceptable salt that is simple to manufacture, bioavailable, cost effective, stable and possesses good pharmacotechnical properties.

Orally disintegrating tablets (ODTs) differ from traditional tablets in that they are designed to be dissolved on the tongue rather than swallowed whole. When administered, an in-situ suspension is created in the oral cavity as the tablet disintegrates, usually in a matter of seconds, and is subsequently swallowed. Orally disintegrating tablets combine the advantages of solid dosage forms with those of liquid forms and appeal to broader groups of patients, i.e. paediatric and geriatric patients who have difficulty in swallowing or chewing solid dosage forms; patients who are unwilling to take solid preparation due to fear of choking; patients with persistent nausea or institutionalized patients.

The performance of orally disintegrating tablets depends on the technology used during their manufacture. The necessary property of such tablets is the ability to disintegrate rapidly and disperse or dissolve in saliva, thereby obviating the need for water. An ideal orally disintegrating tablets does not require water for oral administration yet disintegrates and dissolves in oral cavity within a few seconds; has sufficient strength to withstand the rigors of the manufacturing process and post-manufacturing handling; allows high drug loading; has a pleasant mouth feel; is insensitive to environmental conditions such as humidity and temperature; is adaptable and amenable to existing processing and packaging machineries; is cost-effective.

Orally disintegrating tablets can be produced by application of different technologies, the most common of which are freeze-drying, moulding and standard tabletting. Freeze drying technology has the advantage of immediate dissolution within a few seconds but its use is strongly limited by very poor physical resistance of the final product necessitating the use of special packaging, high cost of production and long processing times. Moulding also provides final dosage forms with very rapid dissolution but with low mechanical strength and high production cost. On the other hand, standard tabletting is the easiest manufacturing process providing the advantages of low production cost since only standard equipment is used as well as common excipients. Orally disintegrating tablets produced by this method are also characterized by satisfactory physical resistance thus there is no need for special packaging as with the other technologies.

High compressible polysaccharides such as maltose, sorbitol, trehalose, maltitol, fructose and especially those having high moldability, are particularly useful in orally disintegrating tablet technology in order to obtain a tablet formulation with adequate hardness that maintains the physical characteristics of the dosage form during production until it comes in contact with moisture such as saliva in mouth, and fast disintegration rate.

Microcrystalline Cellulose, though water insoluble, absorbs water and swells due to capillary action and is an important and very effective excipient in the preparation of orally disintegrating tablets.

It has been surprisingly found that the object of the present invention is achieved when microcrystalline cellulose and maltose are used in a certain ratio. Consequently, the ratio of maltose to microcrystalline cellulose must be in the range of 1:1to 1:4 by weight. Said ratio provides adequate disintegrating properties for the tablets of the present invention but also stabilizes the active pharmaceutical ingredient which does not degrade to unwanted impurities.

Generally, the choice of the disintegrant has a major role in the manufacture of orally disintegrating tablets since it has an impact on both hardness and mouth feel of the composition. Therefore, the choice of a suitable disintegrant and an optimal use level are critical to ensure a high disintegration rate. Some disintegrants, also known as superdisintegrants, are particularly effective in inducing rapid tablet disintegration due to combined effect of swelling and water absorption by the formulation. Due to swelling of superdisintegrants, the wetted surface of the carrier increases thus promotes the wettability and dispersibility of the system and enhances the disintegration and dissolution. Superdisintegrants include, for example, crospovidone, croscarmellose sodium and sodium starch glycolate.

Crospovidone was used for the enhancement of the active ingredient's dissolution rate. Its action relies on a combination of swelling and wicking of liquid into the tablet to generate rapid disintegration. Crospovidone particles are granular and porous compared to other super disintegrants and in combination with their small size provide high surface area. The high surface area combined with unique chemistry results in high interfacial activity and enhances dissolution of poorly soluble drugs, such as Aripiprazole, in a way that is not possible with other disintegrant technologies.

Colloidal silicon has a beneficial effect on the erodibility characteristics of the formulation promoting the disintegration of tablets when combined with another disintegrant. Moreover, Colloidal silicon dioxide contributes in gelling, emulsification and consequently in active ingredient dissolution enhancement.

The pharmaceutical compositions of the present invention are characterized by excellent pharmacotechnical properties, such as flowability, compressibility and homogeneity. In more detail, the solid dosage forms of the present invention exhibit excellent pharmacotechnical characteristics including disintegration times, dissolution rates, hardness, friability, as well as stability.

The in-vitro dissolution studies were conducted according to the FDA-Recommended Dissolution Methods. The tablets of the present invention were tested for dissolution of Aripiprazole in 1000ml of buffer as dissolution media in USP II apparatus and rotated at 75rpm.

The disintegration test determines whether tablets or capsules disintegrate within the prescribed time when placed in a liquid medium. The disintegration test was performed according to European guidelines (European Pharmacopoeia 5.0; 01/2005:20901). The tablets of the present invention disintegrate in less than 2 minutes and most preferably in less than 1 minute in the mouth before swallowing.

The hardness test is intended to determine, under defined conditions, the resistance to crushing of tablets, measured by the force needed to disrupt them by crushing. The test was conducted according to European guidelines (European Pharmacopoeia 5.0; 01/2005:20908). The tablets manufactured in accordance with the present invention have a hardness of less than 100N.

The friability test is intended to determine, under defined conditions, the friability of uncoated tablets, the phenomenon whereby tablet surfaces are damaged and/or show evidence of lamination or breakage when subjected to mechanical shock or attrition. The friability test was performed according to European guidelines (European Pharmacopoeia 5.0; 01/2005:20907).

Moreover, the pharmaceutical compositions of the present invention may also contain one or more additional formulation excipients such as diluents, disintegrants, binders, lubricants, glidants, colorants and flavouring agents, provided that they are compatible with the active ingredient of the composition, so that they do not interfere with it in the composition and in order to increase the stability of the drug and the self-life of the pharmaceutical product.

Diluents may be, for example, microcrystalline cellulose, dextrates, dextrose, fructose, mannitol, sorbitol, starch, pregelatinized starch, sucrose, xylitol, maltose,maltodextrin, maltitol.

Disintegrants may be selected from alginic acid, carbon dioxide, carboxymethylcellulose calcium,carboxymethylcellulose sodium, croscarmellose sodium, guar gum, methylcellulose, polacrilin potassium, poloxamer, sodium alginate, crospovidone.

Binders may be, for example, alginic acid, carbomer, ethyl cellulose, gelatine, liquid glucose, guar gum, hydroxyethyl cellulose, methylcellulose, polydextrose, polyethylene oxide. Also, at least a lubricant is incorporated into the formulation to prevent the powder from adhering to tablet punches during the compression procedure. Lubricants may be, for example, talc, magnesium stearate, calcium stearate, glycerylbehenate, hydrogenated castor oil, stearic acid, sodium lauryl sulphate.

Glidants are used to promote powder flow by reducing interparticle friction and cohesion. These are used in combination with lubricants as they have no ability to reduce die wall friction. Glidants may be, for example, colloidal silicon dioxide, calcium silicate, calcium phosphate tribasic.

Flavouring and sweetening agents are used to mask potential unpleasant tasting active ingredients and improve the likelihood that the patient will complete a course of medication. Flavouring agents may be, for example, mint powder, menthol, cherry flavour, xylitol, vanillin, aspartame, acesulfame potassium, saccharin.

The colorants may include pigments, natural food colours and dyes suitable for food, drug and cosmetic applications. Colorants may be, for example, iron oxides, indigo carmine, sunset yellow FCF.

A number of orodispersible tablets comprising different excipients were tested as presented in the following examples to achieve the optimal properties with respect to the objectives of the present invention.

### EXAMPLES

### Example 1:

**Table 1: Formulations of example 1 (formulations 2-4 are not in the scope of the claims)**

| **Ingredients** | **Formulation** | | | |
|---|---|---|---|---|
| | **1** | **2** | **3** | **4** |
| **Internal Phase** | % **content/tablet** | | | |
| Aripiprazole | 10,00 | 10,00 | 10,00 | 10,00 |
| Maltose | 21,60 | - | - | - |
| Mannitol | - | 21,60 | - | - |
| Glucose | - | - | 21,60 | - |
| Dextrates | - | - | - | 21,60 |
| Crospovidone | 4,00 | 4,00 | 4,00 | 4,00 |
| Microcrystalline Cellulose | 52,15 | 52,15 | 52,15 | 52,15 |
| Colloidal Silicon Dioxide | 2,00 | 2,00 | 2,00 | 2,00 |

| **External Phase** | | | | |
|---|---|---|---|---|
| Croscarmellose Sodium | 3,00 | 3,00 | 3,00 | 3,00 |
| Xylitol 200 | 2,00 | 2,00 | 2,00 | 2,00 |
| Aspartame | 2,00 | 2,00 | 2,00 | 2,00 |
| Cherry Flavour | 0,15 | 0,15 | 0,15 | 0,15 |
| Acesulfame K | 2,00 | 2,00 | 2,00 | 2,00 |
| Iron Oxide Red | 0,10 | 0,10 | 0,10 | 0,10 |
| Mg Stearate | 1,00 | 1,00 | 1,00 | 1,00 |
| **Total for uncoated tablet** | **100,00** | **100,00** | **100,00** | **100,00** |

Four different formulations were prepared in order to identify the influence of the water soluble excipient in the dissolution of the orally disintegrating tablets of the present invention.

The manufacturing process for each tablet of formulations 1 to 4 was:
- The materials of the internal and external phase are weighed and sieved.
- The ingredients of the internal phase are mixed in blender. Blending is performed until uniformity of the powder.
- The granulation liquid, water, is added to the mixture obtained from the previous step.
- The wetted mass is dried at 40°C.
- The granules are sifted through sieve.
- The mixture resulted from the previous step is mixed with the excipients of the external phase.
- The powder mixture is compressed into tablets.

The dissolution rate of the drug from the four formulations was studied, by using USP II apparatus (paddles) at 75 rpm and 1000ml buffer solution with pH 4.

**Table 2: Dissolution results of formulations of example 1**

| **Time (min)** | **% Release** | | | |
|---|---|---|---|---|
| | **Formulation** | | | |
| | **1** | **2** | **3** | **4** |
| 5 | 59,15 | 64,89 | 64,86 | 66,58 |
| 10 | 77,05 | 80,98 | 79,61 | 81,67 |
| 15 | 84,08 | 88,33 | 84,03 | 87,41 |
| 30 | 92,82 | 93,07 | 87,51 | 91,58 |
| 45 | 95,43 | 94,89 | 89,08 | 92,30 |

It is observed that the dissolution profile does not vary a lot between the formulations when changing the water soluble excipient.

Tablets of Formulations 1 to 4 as above were loaded to the stability chambers and monitored, in order to compare their chemical stability. Stability data after 6 months under long term, intermediate and accelerated conditions are presented in the following table.

**Table 3: Stability data of Aripiprazole ODT formulations of example 1**

| **Impurities/Limits** | **Stability data after 6 months** | | | | | | | | | | | |
|---|---|---|---|---|---|---|---|---|---|---|---|---|
| | 25°C/60% RH | | | | 30°C/60% RH | | | | 40°C/75% RH | | | |
| | 'Formulation' | | | | 'Formulation' | | | | 'Formulation' | | | |
| | 1 | 2 | 3 | 4 | 1 | 2 | 3 | 4 | 1 | 2 | 3 | 4 |
| Imp A (NMT 0.5%) | ND | ND | ND | ND | ND | ND | ND | ND | ND | 0.02 | 0.02 | 0.04 |
| Imp B (NMT 0.5%) | ND | ND | ND | ND | ND | ND | ND | ND | ND | ND | ND | 0.03 |
| Imp C (NMT 0.5%) | 0.05 | 0.12 | 0.12 | 0.17 | 0.06 | 0.13 | 0.16 | 0.22 | 0.16 | 0.27 | 0.48 | 0.36 |
| Imp D (NMT 0.5%) | ND | ND | ND | ND | ND | ND | ND | ND | 0.07 | ND | ND | ND |
| Imp E (NMT 0.5%) | ND | ND | ND | ND | ND | ND | ND | ND | ND | ND | ND | ND |
| Imp F (NMT 0.5%) | ND | ND | ND | ND | ND | ND | ND | ND | ND | ND | ND | ND |
| Imp G (NMT 0.5%) | ND | ND | ND | ND | ND | ND | ND | ND | ND | ND | 0.06 | 0.02 |
| Unknown (NMT 0.2%) | 0.14 | 0.08 | 0.08 | 0.12 | 0.14 | 0.09 | 0.08 | 0.10 | 0.14 | 0.13 | 0.39 | 0.70 |
| Total (NMT 2.0%) | 0.19 | 0.20 | 0.20 | 0.29 | 0.20 | 0.22 | 0.24 | 0.32 | 0.37 | 0.42 | 0.89 | 1.15 |

Taking into account the above stability data is clear that the compositions containing maltose and mannitol are the most stable under all tested conditions (especially formulation 1 with maltose as water soluble excipient).

### Example 2:

In order to investigate how the ratio Maltose / Microcrystalline Cellulose would influence the dissolution and the physicochemical properties of the tablets, two new formulations were prepared, one by increasing and one by decreasing this ratio, compared to Formulation 1 of example 1.

**Table 4: Formulation of example 2**

| **Ingredients** | **Formulation** | | |
|---|---|---|---|
| | **1** | **2** | **3** |
| **Internal Phase** | **% content/tablet** | | |
| Aripiprazole | 10,00 | 10,00 | 10,00 |
| Maltose | 21,60 | 11,60 | 31,60 |
| Crospovidone | 4,00 | 4,00 | 4,00 |
| Microcrystalline Cellulose | 52,15 | 62,15 | 42,15 |
| Colloidal Silicon Dioxide | 2,00 | 2,00 | 2,00 |

| **External Phase** | | | |
|---|---|---|---|
| Croscarmellose Sodium | 3,00 | 3,00 | 3,00 |
| Xylitol 200 | 2,00 | 2,00 | 2,00 |
| Aspartame | 2,00 | 2,00 | 2,00 |
| Cherry Flavour | 0,15 | 0,15 | 0,15 |
| Acesulfame K | 2,00 | 2,00 | 2,00 |
| Iron Oxide Red | 0,10 | 0,10 | 0,10 |
| Mg Stearate | 1,00 | 1,00 | 1,00 |
| **Total for uncoated tablet** | **100,00** | **100,00** | **100,00** |

The tablets were prepared with the same manufacturing process as in example 1.

The dissolution rate of the drug was studied, by using USP II apparatus (paddles) at 75 rpm and 1000ml buffer solution with pH 4.

**Table 5: Dissolution results of formulations of example 2**

| **Time (min)** | **% Release** | | |
|---|---|---|---|
| | **Formulations** | | |
| | **1** | **2** | **3** |
| 5 | 59,15 | 26,41 | 61,71 |
| 10 | 77,05 | 43,80 | 81,89 |
| 15 | 84,08 | 57,33 | 87,87 |
| 30 | 92,82 | 65,86 | 94,92 |
| 45 | 95,43 | 74,98 | 95,92 |

Comparing the % drug release of Formulations 2 and 3 of Example 2 with Formulation 1 of Example 1 it can be concluded that Formulation 3 of Example 2 has comparable dissolution profile with Formulation 1 of Example 1. This indicates that the target dissolution profile is achieved when the ratio of maltose to microcrystalline cellulose is in the range of 1:1 to 1:4 by weight.

### Example 3

In order to improve the pharmacotechnical properties of the tablets of Formulation 1 according to Examples 1 and 2 the content of colloidal silicon dioxide was increased at the expense of Maltose and Microcrystalline Cellulose. This had as a result the disintegration time to remain short, even when compressing the tablets with higher compaction force.

The preferred orodispersible tablet composition according to the present invention is illustrated in Table 6 below:

**Table 6: Formulation of example 3**

| **Ingredients** | **% content/tablet** |
|---|---|
| **Internal Phase** | |
| Aripiprazole | 10,00 |
| Maltose | 16,60 |
| Crospovidone | 4,00 |
| Microcrystalline Cellulose | 40,23 |
| Colloidal Silicon Dioxide | 5,00 |

| **External Phase** | |
|---|---|
| Croscarmellose Sodium | 6,50 |
| Crospovidone | 6,50 |
| Xylitol 200 | 1,00 |
| Aspartame | 2,00 |
| Cherry Flavour | 0,15 |
| Acesulfame K | 2,00 |
| Iron Oxide Red/Yellow | 0,02 |
| Talc | 4,00 |
| Sodium stearyl fumarate | 2,00 |
| **Total for uncoated tablet** | **100,00** |

The manufacturing process of tablets of example 3 according to the present invention comprises the following steps:
- The materials of the internal and external phase are weighed and sieved.
- The ingredients of the internal phase are mixed in blender. Blending is performed until uniformity of the powder.
- The granulation liquid, water, is added to the mixture obtained from the previous step.
- The wetted mass is dried at 40°C.
- The granules are sifted through sieve.
- The mixture resulted from the previous step is mixed with the excipients of the external phase.
- The powder mixture is compressed into tablets.

The dissolution rate of the drug was studied, by using USP II apparatus (paddles) at 75 rpm and 1000ml buffer solution with pH 4.

**Table 7: Dissolution results of example 3**

| **Time (min)** | **% Release** |
|---|---|
| | **Example 3** |
| 5 | 57,73 |
| 10 | 69,83 |
| 15 | 75,65 |
| 30 | 85,05 |
| 45 | 90,44 |

The dissolution profile given from the dissolution test of the orodispersible tablets of example 3 was the desirable. Stability data were performed according to ICH guidelines in all storage conditions and the results were well within the specifications.

## Claims

1. An orally dispersible pharmaceutical composition comprising Aripiprazole or a pharmaceutical acceptable salt thereof as the active ingredient and an effective amount of maltose and microcrystalline cellulose wherein the ratio of maltose to microcrystalline cellulose is in the range of 1:1 to 1:4 by weight.

2. The pharmaceutical composition according to claim 1, wherein it further comprises Crospovidone as a disintegrant.

3. The pharmaceutical composition according to claim 2, wherein Crospovidone is added both extragranurarly and intragranurarly.

4. The pharmaceutical composition according to any of claims 1 to 3, wherein it further comprises at least one pharmaceutically acceptable excipient selected from glidants, lubricants and flavouring agents.

5. A process for the preparation of an orally dispersible pharmaceutical composition comprising Aripiprazole or a pharmaceutical acceptable salt thereof as an active ingredient and an effective amount of maltose and microcrystalline cellulose wherein the ratio of maltose to microcrystalline cellulose is in the range of 1:1 to 1:4 by weight, which comprises:
- Weighing the active ingredient and the excipients of internal and external phase and sieving;
- Blending the active ingredient with at least maltose, microcrystalline cellulose, a part of crospovidone and the rest of the excipients of internal phase until complete homogeneity.
- Kneading the above mixture with water and the drying the wetted mass.
- Sieving the dried mass and adding to the sieved mixture Crospovidone and the remaining external phase excipients and mixing until uniformity.
- Compressing the resulted mixture into a tablet dosage form.

## Patentansprüche

1. Oral dispergierbare pharmazeutische Zusammensetzung umfassend Aripiprazol oder ein pharmazeutisch verträgliches Salz davon als Wirkstoff und eine wirksame menge Maltose und mikrokristalline Cellulose, wobei das Verhältnis von Maltose zu mikrokristalliner Cellulose im bereich von 1: 1 bis 1: 4 Gewicht liegt.

2. Pharmazeutische Zusammensetzung nach Anspruch 1, wobei sie ferner Crospovidon als Sprengmittel enthält.

3. Pharmazeutische Zusammensetzung nach Anspruch 2, wobei Crospovidon sowohl extragranular als auch intragranular zugegeben wird.

4. Pharmazeutische Zusammensetzung nach einem der Ansprüche 1 bis 3, wobei sie ferner mindestens einen pharmazeutisch akzeptablen Hilfsstoff umfasst, das aus Gleitmitteln, Schmiermitteln und Geschmacksstoffen ausgewählt ist.

5. Verfahren zur Herstellung einer oral dispergierbaren pharmazeutische Zusammensetzung umfassend Aripiprazol oder ein pharmazeutisch verträgliches Salz davon als Wirkstoff und eine wirksame menge Maltose und mikrokristalline Cellulose, wobei das Verhältnis von Maltose zu mikrokristalliner Cellulose im Bereich von 1: 1 bis 1: 4 nach Gewicht liegt, bestehend aus:
- des Wirkstoffs und der Hilfsstoffe der inneren und äußeren Phase Abwägen und anschließendes sieben;
- des Wirkstoffs mit mindestens Maltose, mikrokristalliner Cellulose, einem Teil von Crospovidon und den übrigen Hilfsstoffen der inneren Phase bis zur vollständigen Homogenität mischen;
- der obigen Mischung mit Wasser kneten und der benetzten Masse trocknen;
- der getrockneten Masse sieben und zu der gesiebten Mischung aus Crospovidon und den restlichen Hilfsstoffen der äußeren Phase zugeben und dann bis zur Gleichförmigkeit mischen;
- der resultierenden Mischung in eine Tabletten-Dosierungsform komprimieren.

## Revendications

1. Une composition pharmaceutique oralement dispersible comprenant l'Aripiprazole ou un sel pharmaceutiquement acceptable de celui-ci comme principe actif et une quantité efficace de maltose et de cellulose microcristalline où le rapport du maltose à la cellulose microcristalline est dans l'intervalle de 1:1 à 1:4 en poids.

2. La composition pharmaceutique selon la revendication 1, dans laquelle est comprise en outre la Crospovidone comme désintégrant.

3. La composition pharmaceutique selon la revendication 2, dans laquelle la Crospovidone est ajoutée à la fois extragranulairement et intragranulairement.

4. La composition pharmaceutique selon l'une quelconque des revendications 1 à 3, dans laquelle est compris en outre au moins un excipient pharmaceutiquement acceptable sélectionné parmi des agents de glissement, des lubréfiants et des agents aromatisants.

5. Un procédé de préparation d'une composition pharmaceutique oralement dispersible comprenant l'Aripiprazole ou un sel pharmaceutiquement acceptable de celui-ci comme principe actif et une quantité efficace de maltose et de cellulose microcristalline où le rapport du maltose à la cellulose microcristalline est dans l'intervalle de 1:1 à 1:4 en poids, qui comprend:
- peser le principe actif et les excipients des phases interne et externe et tamiser;
- combiner le principe actif avec au moins le maltose, la cellulose microcristalline, une part de Crospovidone et le reste d'excipients de la phase interne jusqu'à homogénéité complète;
- malaxer le mélange ci-dessus avec de l'eau et sécher la masse mouillée;
- tamiser la masse séchée et ajouter au mélange tamisé la Crospovidone et le reste d'excipients de la phase externe et mélanger jusqu'à uniformité;
- compresser le mélange resultant sous forme posologique de comprimés.
